# EUROPEAN PATENT APPLICATION

(11) **EP 4 846 989 A1**
(43) Date of publication of application: **02.12.2026**
(21) Application number: 26181345.5
(22) Date of filing: 27.05.2026
(51) Int. Cl.: A61F 11/06, A61F 9/02, B22F 1/16, H01F 1/06, A61L 31/00

(54) **PROTECTIVE EQUIPMENT CONTAINING MAGNETIC METALLIC PARTICLES AND MANUFACTURING PROCESS THEREOF**

(30) Priority: 27.05.2025 US 202563812440 P
(71) Applicant: Alan-Safety Co., Ltd, Tainan (TW)
(72) Inventor: TSAI, Ming-Hsiang, Tainan (TW)
(74) Representative: Epping - Hermann - Fischer

(57) **Abstract**

A metal detectable protective equipment and a manufacturing process thereof. The protective equipment comprises: a body formed by a polymeric material, and magnetic metallic particles dispersed in the body, wherein the magnetic metallic particle has a core-shell structure and the core consists essentially of a ferrimagnetic material.

## Description

### BACKGROUND OF THE INVENTION

### 1. Field of the Invention

The present invention relates to a personal protective equipment, and more particularly to a personal protective equipment detectable by a metal detector.

### 2. Description of the Related Art

Personal protective equipment (PPE) protects a user from exposure to harmful conditions such as noise, pollutants, and hazardous substances. In factories, the production process is often accompanied by noise and dust. Workers must wear the PPE such as protective eyewear and earplugs to ensure safety in such working environment.

In manufacturing process of many products such as food or medical products, metal detectors are typically located at the end of the production line to detect whether products contain contaminants or foreign objects. However, if the PPE such as protective eyewear or earplug enters the production line, the metal detector cannot be able to detect their presence because of its non-metallic materials, causing a risk to product control.

In order to make the PPE detectable, it has been reported that metal wires or metal rods are set in the PPE. For example, US Pat. No. 11,690,762 discloses the metal-detectable insert is disposed in the protective eyewear. For example, the safety earplug contains a metal component in its main body and/or connecting wire. However, this arrangement increases the difficulty of manufacturing process of the PPE. The obtained protective eyewear or earplug can easily cause discomfort to the user when wearing them.

Earplug and safety glasses are generally made of resin materials. It also has been reported in US Pat. No. 8,342,679 that metal particles are added into the resin materials to prepare safety glasses containing metal particles. However, metals themselves easily react with resin materials during the manufacturing process. Another problem is that metals may undergo oxidation when exposed to air. Because of the above reactions of metals, the earplugs and safety glasses containing metal particles may become undetectable.

Therefore, there is still a need for personal protective equipment detectable by metal detector.

### SUMMARY

The present application provides a protective equipment comprising: a body formed by a polymeric material, and magnetic metallic particles dispersed in the body, wherein the magnetic metallic particle has a core-shell structure and the core consists essentially of a ferrimagnetic material.

In some embodiments, the protective equipment is an earplug, which comprises: a body formed by thermoplastic rubber (TPR) and/or polyurethane (PU), magnetic metallic particles having a core-shell structure, wherein the core consists essentially of magnetite (Fe₃O₄) and the shell comprises polystyrene, and the magnetic metallic particles is 5~15 %, based on the total weight of the earplug.

In other embodiments, the protective equipment is a protective eyewear, which comprises: a body formed by the polymeric material selected from a group consisting of thermoplastic rubber (TPR), polyvinyl chloride (PVC), polycarbonate (PC), polyamide (PA), and polypropylene (PP), magnetic metallic particles having a core-shell structure, wherein the core consists essentially of magnetite (Fe₃O₄) and the shell comprises polystyrene, and the magnetic metallic particles is 5~15 %, based on the total weight of the protective eyewear.

The present application also provides a process for preparing the protective equipment comprising: mixing a polymeric material and magnetic metallic particles to form a mixture, and molding the mixture.

By applying the magnetic metallic particle, the protective equipment of the present application can be detected by the metal detector. Simultaneously, the present application improves chemical stability, reduces difficulty of manufacturing process and eliminates the user wearing discomfort.

### BRIEF DESCRIPTION OF THE DRAWINGS

None.

### DETAILED DESCRIPTION OF THE EMBODIMENTS

The present application provides a metal detectable protective equipment with improvements in chemical stability, process simplicity and user comfort. The protective equipment comprises: a body formed by a polymeric material, and magnetic metallic particles dispersed in the body.

The present application also provides a process for preparing the protective equipment comprising: mixing a polymeric material and magnetic metallic particles to form a mixture, and molding the mixture. The process can further comprise steps including dispersing the magnetic metallic particles in the mixture, adding metal particles and/or an additive to the mixture, heating the mixture, maintaining the temperature of the mixture, releasing the molded product, processing the molded product and the like.

In the present application, the protective equipment can include an earplug, a protective eyewear, a mask, a face shield, and the like.

In some embodiments, the magnetic metallic particles are uniformly dispersed in the body, and the body constitutes the main structure of the protective equipment. For example, the protective equipment can include a pair of earplugs and a connecting cable therebetween. In one embodiment, the body can merely include the earplug. In one embodiment, the body can include the earplugs and the connecting cable. For example, the protective equipment can be a protective eyewear comprising lenses and frames. In one embodiment, the body can merely include the frame. In one embodiment, the body can include the frames and the lenses.

The polymeric material forming the body is not particularly limited and can include the polymeric materials conventionally applied to manufacture of protective equipment. In embodiments, the polymeric material can include thermoplastic rubber (TPR), polyurethane (PU), polyvinyl chloride (PVC), polycarbonate (PC), polyamide (PA), polypropylene (PP), or any combination thereof.

In the present application, the magnetic metallic particles have a core-shell structure. The core is formed by a ferrimagnetic material, and the shell is formed by a polymer.

The ferrimagnetic material means substances with antiparallel magnetic moments of different magnitudes, resulting in a net spontaneous magnetization. In one embodiment, suitable ferrimagnetic material can include magnetite (Fe₃O₄) and ferrites such as yttrium iron garnet (YIG), nickel ferrite (NiFe₂O₄), barium hexaferrite (Ba Fe₁₂O₁₉) and the like. In preferred embodiments, the ferrimagnetic material is magnetite (Fe₃O₄).

The polymer is used to form the shell surrounding the ferrimagnetic core. The polymer shell shields the internal ferrimagnetic core to prevent the core from oxidizing or deteriorating due to exposure to ambient environment, such that the protective equipment containing the magnetic metallic particles can maintain the metal-detectable properties for a longer period of time. The polymer is not particularly limited, as long as it does not affect the metal-detectable properties of the core. Example of suitable polymer includes polystyrene, polyacrylic resins such as poly(methyl methacrylate), acrylonitrile-styrene copolymers, styrene-butadiene-styrene block copolymer and the like.

In embodiments, the magnetic metallic particles have a core-shell structure, the core consists essentially of magnetite (Fe₃O₄) and the shell comprises polystyrene. In one embodiment, the core is magnetite (Fe₃O₄) and the shell is polystyrene.

The magnetic metallic particles have an average particle diameter of 300~500 nm. In embodiments, the particle diameter can be 300 nm, 350 nm, 400 nm, 450 nm, 500 nm or a value falling within the scope between any two of the above values. In the preferred embodiments, the particle diameter is 300~400 nm.

The magnetic metallic particles have a true density of 1.0~2.5 g/cm³. In embodiments, the true density can be 1.0 g/cm³, 1.2 g/cm³, 1.4 g/cm³, 1.5 g/cm³, 1.6 g/cm³, 1.8 g/cm³, 1.9 g/cm³, 2.0 g/cm³, 2.2 g/cm³, 2.5 g/cm³, or a value falling within the scope between any two of the above values. In the preferred embodiments, the true density is 1.5~1.9 g/cm³.

The magnetic metallic particles have a bulk density of 0.1~1.0 g/cm³. In embodiments, the bulk density can be 0.1 g/cm³, 0.2 g/cm³, 0.3 g/cm³, 0.4 g/cm³, 0.5 g/cm³, 0.6 g/cm³, 0.7 g/cm³, 0.8 g/cm³, 0.9 g/cm³, 1.0 g/cm³, or a value falling within the scope between any two of the above values. In the preferred embodiments, the bulk density is 0.55~0.75 g/cm³.

The amount of the magnetic metallic particle can be, based on the total weight of the protective equipment, 5~15 wt%, such as 5 wt%, 7.5 wt%, 10 wt%, 12.5 %, 15 wt% or a value falling within the scope between any two of the above values.

The core-shell structure of the magnetic metallic particle facilitates easy and uniform dispersion of the particles in various polymeric materials such as resins and engineering plastics. If the amount of the magnetic metallic particle exceeds the upper limit, it will adversely affect the material properties such as hardening or embrittlement. The excessive amount can also cause process difficulties, such as problems of particle dispersion. If the amount of the magnetic metallic particle is below the lower limit, the protective equipment has insufficient metal-detectable properties, i.e. the protective equipment may not be detectable when passing through the metal coil detection gate.

The magnetic metallic particles having core-shell structure can be prepared by the conventional preparation methods of core-shell metallic particles, which is not limited in the present application. For example, the ferrimagnetic core can be coated with polymer such as polystyrene to form the desired structure.

In one embodiment, the protective equipment is an earplug, the body is formed by thermoplastic rubber (TPR) and/or polyurethane (PU), the magnetic metallic particle has a core-shell structure, the core consists essentially of magnetite (Fe₃O₄) and the shell comprises polystyrene, and the magnetic metallic particles is 5~15 %, based on the total weight of the protective equipment.

In one embodiment, the protective equipment is a protective eyewear, the body is formed by the polymeric material selected from a group consisting of thermoplastic rubber (TPR), polyvinyl chloride (PVC), polycarbonate (PC), polyamide (PA), and polypropylene (PP), the magnetic metallic particle has a core-shell structure, the core consists essentially of magnetite (Fe₃O₄) and the shell comprises polystyrene, and the magnetic metallic particles is 5~15 %, based on the total weight of the protective equipment.

In some embodiments, in addition to the magnetic metallic particles, the protective equipment further comprises metal particles. The metal can include, but not limited to, iron, nickel, cobalt, zinc, manganese, aluminum, copper, and the like. In some embodiments, the metal particles have a core-shell structure. The structure can be metallic core and polymer shell.

The protective equipment of the present application can be detected by the metal detector. The metal detector can include any type of metal detector that is capable of detecting the protective equipment, for example, the metal detector commonly used in the food industry, the pharmaceutical industry, or other manufacturing industries.

In one embodiment, the protective equipment is detectable by metallic sensor coils. Electromagnetic induction is applied to the metallic sensor coils. A magnetic field can be generated by an electric current passing through the metallic sensor coils. When the magnetite material such as Fe₃O₄ passes through the metallic sensor coils, eddy currents are generated and then change the original magnetic field of the metallic coils. The change of magnetic field can be detected by the sensor. Accordingly, the protective equipment containing the magnetic metallic particles can be detected by the metallic sensor coils.

According to the above, by applying the magnetic metallic particle, the protective equipment of the present application can be detected by the metal detector. The core-shell structure of the magnetic metallic particle prevents the core from oxidization or deterioration, improves chemical stability, and maintains the metal-detectable properties for a longer period of time.

By incorporating the magnetic metallic particles into the polymeric material, there is no need to separately configure metal wires or metal rods in the protective equipment. It eliminates the user wearing discomfort. Moreover, the protective equipment can be manufactured by the general process except an extra step of adding the magnetic metallic particles. Therefore, the manufacturing process can be simplified.

### EXAMPLES

The magnetite (Fe₃O₄) particles used in the following examples are core-shell particles, which have an average particle diameter of 350 nm, a true density of 1.55~1.82 g/cm³, and a bulk density of 0.61 g/cm³. The magnetite is easily attracted by magnets, and is more likely to interfere with the magnetic field when passing through the metallic sensor coils. Furthermore, the magnetite possesses chemical stability. The magnetite powder has an original average particle diameter of 300 nm. After the polystyrene coating step, the obtained core-shell particles have an average particle diameter of 350 nm.

The exemplified compositions for the earplug or the safety glasses can include:
(1) PU Earplug: polyisocyanate, water-based acrylic emulsion, and magnetite particles.
(2) PU Earplug: polyisocyanate, polyether polyol mixture, water, foam stabilizer, catalyst, and magnetite particles.
(3) TPR Earplug: thermoplastic rubber (TPR) and magnetite particles.
(4) TPR safety glasses (frames): thermoplastic rubber (TPR), compatibilizer, and magnetite particles.
(5) PC safety glasses (frames): polycarbonate (PC), compatibilizer, and magnetite particles.
(6) PVC safety glasses (frames): polyvinyl chloride (PVC), compatibilizer, and magnetite particles.
(7) PA/Nylon safety glasses (frames): polyamide (PA, Nylon), compatibilizer, and magnetite particles.
(8) PP safety glasses (frames): polypropylene (PP), compatibilizer, and magnetite particles.

### Example 1

### PU earplugs

100 parts by weight of polyisocyanate was fed to a tank and the temperature was maintained at 38-42°C with stirring. 100 parts by weight of aqueous acrylic emulsion was mixed with 10 parts, 15 parts, 20 parts and 30 parts of the magnetite particles, respectively, and dispersed at 2,000 rpm for 20 minutes to form a stable mixture emulsion. This emulsion was maintained at 18-25°C with stirring. The polyisocyanate (100 parts) and the emulsion (110 parts, 115 parts, 120 parts and 130 parts, respectively) were mixed under stirring at 3,000 rpm. The mixture was extruded into a mold. After the polyurethane foaming reaction was completed, the product was released from the mold. The products contained, based on the total weight of the product, 5 wt%, 7.5 wt%, 10 wt% and 15 wt% of the magnetite particles respectively.

### Example 2

### PU earplugs

100 parts by weight of NCO-terminated PU prepolymer was fed to a tank and the temperature was maintained at 40-45 °C with stirring. 100 parts by weight of polypropylene glycol mixture (a weight ratio of mw 6,000 PG: mw 400 PG = 60: 40) was mixed with 10 parts, 15 parts, 20 parts, and 30 part of the magnetite particles by polyol blend, respectively, 0.7-19 parts of water, 0.3-1.2 parts of foam stabilizer and 0.5-1.5 parts of catalyst and dispersed at 1,000 rpm for 20 minutes to form a stable mixture. This mixture was maintained at 40-45°C with stirring. The NCO-terminated PU prepolymer (100 parts) and the mixture (110-130 parts) were mixed under stirring at 3,000 rpm, and then was extruded into a mold. After the polyurethane foaming reaction was completed, the product was released from the mold. The products contained, based on the total weight of the product, 5, 7.5, 10 and 15 wt% of the magnetite particles respectively.

### Example 3

### TPR Earplug

100 parts by weight of styrene-butadiene rubber and 5 parts, 10 parts, or 15 parts by weight of the magnetite particles, respectively, were fed into a mixer to knead and disperse, and then injected into a mold. After cooling, the product was released from the mold. The products contained, based on the total weight of the product, 5, 10, 15 wt% of the magnetite particles respectively.

### Example 4

### TPR safety glasses (frames)

100 parts by weight of styrene-butadiene rubber and 5 parts, 10 parts, or 15 parts by weight of the magnetite particles were fed into a mixer to knead and disperse, and then injected into a mold. After cooling, the product was released from the mold. The products contained, based on the total weight of the product, 5, 10, and 15 wt% of the magnetite particles respectively.

### Example 5

### PC safety glasses (frames)

100 parts by weight of polycarbonate, 5 parts, 10 parts, or 15 parts by weight of the magnetite particles and 2-5 parts of graft-modified polycarbonate compatibilizer were fed into a mixer to knead and disperse, and then injected into a mold. After cooling, the product was released from the mold. The products contained, based on the total weight of the product, 5, 10, and 15 wt% of the magnetite particles respectively.

### Example 6

### PVC safety glasses (frames)

100 parts by weight of polyvinyl chloride (PVC), 5 parts, 10 parts, or 15 parts by weight of the magnetite particles were fed into a mixer to knead and disperse, and then injected into a mold. After cooling, the product was released from the mold. The products contained, based on the total weight of the product, 5, 10, and 15 wt% of the magnetite particles respectively.

### Example 7

### PA/Nylon safety glasses (frames)

100 parts by weight of polyamide (PA, Nylon), 5 parts, 10 parts, or 15 parts by weight of the magnetite particles and 4-7 parts of graft-modified polycarbonate compatibilizer were fed into a mixer to knead and disperse, and then injected into a mold. After cooling, the product was released from the mold. The products contained, based on the total weight of the product, 5, 10, and 15 wt% of the magnetite particles respectively.

### Example 8

### PP safety glasses (frames)

100 parts by weight of polypropylene (PP), 5 parts, 10 parts, or 15 parts by weight of the magnetite particles were fed into a mixer to knead and disperse, and then injected into a mold. After cooling, the product was released from the mold. The products contained, based on the total weight of the product, 5, 10, and 15 wt% of the magnetite particles respectively.

### Comparative Examples 1-8

The control group followed the same steps as in the above Examples 1-8, but without adding the magnetite particles.

### Test Example 1

### Metal-detectable Property Test

The products of Examples 1-8 and Comparative Examples 1-8 were passed through a metal detector. The results were shown in the Table 1.

**Table 1**

| Example | Content of the magnetite particles (wt %) | Millivolt (mv) |
|---|---|---|
| Example 1 | 5 | 1,361 |
| | 7.5 | 2,127 |
| | 10 | 2,509 |
| | 15 | 4,281 |
| Comparative Example 1 | 0 | 0 |
| Example 2 | 5 | 1,418 |
| | 7.5 | 2,153 |
| | 10 | 2,601 |
| | 15 | 4,354 |
| Comparative Example 2 | 0 | 0 |
| Example 3 | 5 | 1,484 |
| | 10 | 2,164 |
| | 15 | 2,750 |
| Comparative Example 3 | 0 | 0 |
| Example 4 | 5 | 13,895 |
| | 10 | 14,904 |
| | 15 | 21,179 |
| Comparative Example 4 | 0 | 0 |
| Example 5 | 5 | 15,666 |
| | 10 | 17,533 |
| | 15 | 33,156 |
| Comparative Example 5 | 0 | 0 |
| Example 6 | 5 | 14,615 |
| | 10 | 15,874 |
| | 15 | 34,153 |
| Comparative Example 6 | 0 | 0 |
| Example 7 | 5 | 13,454 |
| | 10 | 14,912 |
| | 15 | 21,533 |
| Comparative Example 7 | 0 | 0 |
| Example 8 | 5 | 12,062 |
| | 10 | 13,752 |
| | 15 | 19,427 |
| Comparative Example 8 | 0 | 0 |

### Test Example 2

### Magnetic Adsorption Test

Magnets with 2,000 to 4,000 milligauss were used to verify the magnetic effect of the products of Example 1, Example 2, Comparative Example 1, and Comparative Example 2. The results were shown in Table 2.

**Table 2**

| Example | Content of the magnetite particles (wt %) | Magnetic Adsorption |
|---|---|---|
| Example 1 | 5 | ○ |
| | 7.5 | ○ |
| | 10 | ○ |
| | 15 | ○ |
| Comparative Example 1 | 0 | x |
| Example 2 | 5 | ○ |
| | 7.5 | ○ |
| | 10 | ○ |
| | 15 | ○ |
| Comparative Example 2 | 0 | x |

| | | |
|---|---|---|
| ∘: The product attracted magnets. x: The product did not attract magnets. | | |

Accordingly, the earplugs and the safety glasses of the present application can be detected by the metal detector. The manufacturing process of these products is the same as that of conventional products and just adding the magnetite particles to the raw materials. The magnetite particles possess chemical stability in the products and impart magnetic and metal-detectable properties to the products. In addition, by incorporating the magnetic metallic particles into the raw materials, there is no need to separately configure metal wires or metal rods in the earplugs and the safety glasses. It eliminates the user wearing discomfort.

The core-shell structure of the magnetic metallic particle facilitates easy and uniform dispersion of the particles in the polymeric materials. If the amount of the magnetic metallic particle is too high, it will adversely affect the material properties such as hardening or embrittlement. The excessive amount can also cause process difficulties, such as problems of particle dispersion. As such, the product cannot be molded or the yield of the process will be very low. If the amount of the magnetic metallic particle is too low, the protective equipment has insufficient metal-detectable properties, i.e. the protective equipment may not be detectable when passing through the metal coil detection gate.

While the present invention is disclosed by reference to the preferred embodiments and examples detailed above, it is to be understood that these examples are intended in an illustrative rather than in a limiting sense. It is contemplated that modifications and combinations will readily occur to those skilled in the art, which modifications and combinations will be within the spirit of the invention and the scope of the following claims and its equivalent systems and methods. All features disclosed for the claimed protective equipment apply also to the process for preparing a protective equipment and vice versa.

## Claims

1. A protective equipment comprising:
a body formed by a polymeric material, and
magnetic metallic particles dispersed in the body,
wherein the magnetic metallic particle has a core-shell structure and the core
consists essentially of a ferrimagnetic material.

2. The protective equipment of claim 1, wherein the ferrimagnetic material is selected from a group consisting of magnetite (Fe₃O₄) and ferrites.

3. The protective equipment of claim 1, wherein the magnetic metallic particle has a core-shell structure, the core consists essentially of magnetite (Fe₃O₄) and the shell comprises polystyrene.

4. The protective equipment of claim 1, wherein the magnetic metallic particles have an average particle diameter of 300~350 nm, a true density of 1.0~2.5 g/cm³, and a bulk density of 0.1~1.0 g/cm³.

5. The protective equipment of claim 1, wherein the magnetic metallic particle is 5~30 %, based on the total weight of the protective equipment.

6. The protective equipment of claim 1, further comprising metal particles.

7. The protective equipment of claim 6, wherein the metal is selected from a group consisting of iron, nickel, cobalt, zinc, manganese, aluminum, and copper.

8. The protective equipment of claim 1, wherein the polymeric material is selected from a group consisting of thermoplastic rubber (TPR), polyurethane (PU), polyvinyl chloride (PVC), polycarbonate (PC), polyamide (PA), and polypropylene (PP).

9. The protective equipment of claim 1, which is detectable by metallic sensor coils.

10. The protective equipment of claim 1, which is an earplug, a protective eyewear, a mask or a face shield.

11. The protective equipment of claim 1, wherein
the protective equipment is an earplug,
the body is formed by thermoplastic rubber (TPR) and/or polyurethane (PU),
the magnetic metallic particle has a core-shell structure, the core consists essentially of magnetite (Fe₃O₄) and the shell comprises polystyrene, and
the magnetic metallic particles is 5~15%, based on the total weight of the protective equipment.

12. The protective equipment of claim 1, wherein
the protective equipment is a protective eyewear,
the body is formed by the polymeric material selected from a group consisting of thermoplastic rubber (TPR), polyvinyl chloride (PVC), polycarbonate (PC), polyamide (PA), and polypropylene (PP),
the magnetic metallic particle has a core-shell structure, the core consists essentially of magnetite (Fe₃O₄) and the shell comprises polystyrene, and
the magnetic metallic particles is 5~15%, based on the total weight of the protective equipment.

13. A process for preparing a protective equipment comprising:
mixing a polymeric material and magnetic metallic particles to form a mixture, and
molding the mixture,
wherein the magnetic metallic particle has a core-shell structure and the core consists essentially of a ferrimagnetic material.

14. The process of claim 13, further comprising: dispersing the magnetic metallic particles in the mixture after the mixing step.

15. The process of claim 13, wherein the ferrimagnetic material is selected from a group consisting of magnetite (Fe₃O₄) and ferrites.

16. The process of claim 13, wherein the magnetic metallic particle has a core-shell structure, the core consists essentially of magnetite (Fe₃O₄) and the shell comprises polystyrene.

17. The process of claim 13, wherein the magnetic metallic particles have an average particle diameter of 300~350 nm, a true density of 1.0~2.5 g/cm³, and a bulk density of 0.1~1.0 g/cm³.

18. The process of claim 13, wherein the magnetic metallic particle is 5~15 %, based on the total weight of the protective equipment.

19. The process of claim 13, wherein the polymeric material is selected from a group consisting of thermoplastic rubber (TPR), polyurethane (PU), polyvinyl chloride (PVC), polycarbonate (PC), polyamide (PA), and polypropylene (PP).

20. The process of claim 13, further comprising: adding metal particles into the mixture.
